# EUROPÄISCHE PATENTANMELDUNG

(11) **EP 0 843 995 A2**
(43) Veröffentlichungstag der Anmeldung: **27.05.1998**
(21) Anmeldenummer: 97119488.1
(22) Anmeldetag: 07.11.1997
(51) Int. Cl.: A61K 7/42, C07D 235/20, C07D 413/04, C07D 417/04

(54) **Verwendung von substituierten Benzazolen als UV-Absorber, neue Benzazole und Verfahren zu ihrer Herstellung**

(30) Priorität: 20.11.1996 DE 19648010
(71) Anmelder: HAARMANN & REIMER GMBH, D-37601 Holzminden (DE)
(72) Erfinder: Dilk, Erich, Dr., 37603 Holzminden (DE); Langner, Roland, 37639 Bevern (DE); Johncock, William, Dr., 37649 Heinsen (DE)
(74) Vertreter: Zobel, Manfred, Dr.

(57) **Zusammenfassung**

Die Erfindung betrifft N-substituierte Benzazole, Verfahren zu ihrer Herstellung und ihre Verwendung als UV-Absorber.

## Beschreibung

Die Erfindung betrifft die Verwendung N-substituierter Benzazole als UV-Absorber, neue N-substituierte Benzazole und Verfahren zu ihrer Herstellung.

UV-Absorber sind Verbindungen mit einem ausgeprägten Absorptionsvermögen für die Ultraviolettstrahlung. Sie werden insbesondere als Sonnenschutzmittel in kosmetischen und pharmazeutischen Präparaten, aber auch zur Verbesserung der Lichtbeständigkeit von technischen Produkten, wie Anstrichen, Lacken, Kunststoffen, Textilien, Verpackungsmaterialien und Kautschuken verwendet.

UV-Strahlen werden je nach Wellenlänge in UV-A-Strahlen (320-400 nm, UV-A-I: 340-400 nm, UV-A-II: 320-340 nm) oder UV-B-Strahlen (280-320 nm) eingeteilt. Ganz allgemein gilt: Die schädigende Wirkung der UV-Strahlen auf die menschliche Haut steigt mit sinkender Wellenlänge und steigender Dauer der Exposition.

UV-Strahlen können akute und chronische Hautschädigungen bewirken, wobei die Art der Schädigung von der Wellenlänge der Strahlung abhängt. So kann die UV-B-Strahlung einen Sonnenbrand (Erythem) bis hin zu schwersten Hautverbrennungen verursachen. Auch Minderungen von Enzymaktivitäten, Störungen der DNS-Struktur und Veränderungen an der Zellmembran als schädigende Wirkung der UV-B-Strahlen sind bekannt. Die UV-A-Strahlen dringen in tiefere Hautschichten ein und können dort den Alterungsprozeß der Haut beschleunigen. Die kürzerwellige UV-A-II-Strahlung verstärkt zusätzlich die Bildung von Sonnenbrand. Außerdem kann die UV-A-Strahlung phototoxische oder photoallergische Hautreaktionen auslösen.

Sehr häufige und ungeschützte Bestrahlung der Haut mit Sonnenlicht führt zu einem Verlust der Hautelastizität und zu vermehrter Faltenbildung. In extremen Fällen werden krankhafte Hautveränderungen bis hin zum Hautkrebs beobachtet.

Entsprechend der Lage ihrer Absorptionsmaxima werden UV-Absorber in UV-A- und UV-B-Absorber eingeteilt; wird von einem UV-Absorber sowohl UV-A als auch UV-B absorbiert, spricht man in diesem Fall von einen UV-A/B-Breitbandabsorber.

Es gibt eine Reihe wirksamer UV-B-Absorber, wie z.B. p-Methoxyzimtsäure-2-ethylhexylester, p-Methoxyzimtsäureisoamylester, Phenylbenzimidazol-sulfonsäure (und ihre Salze) und 3-(4'-Methylbenzyliden)-campher.

Die Anzahl geeigneter UV-A-Absorber ist deutlich geringer, und sie weisen erhebliche Mängel auf:

So sind 4-tert-Butyl-methoxy-dibenzoylmethan und 4-Isopropyl-dibenzoylmethan (DE-OS 2 945 925) nicht sehr lichtstabil. Außerdem haben sie nur eine begrenzte Löslichkeit in kosmetischen Ölen, was zu Problemen in der Formulierung kosmetischer Zubereitungen führen kann. Außerdem können Dibenzovlmethanderivathaltige Sonnenschutzprodukte auf Textilien extrem schwer auswaschbare Flecken hinterlassen.

Die als UV-A/B-Breitbandabsorber verwendeten Benzophenone haben nicht die gewünschte breite UV-A- und UV-B-Absorption oder besitzen nur eine geringe Absorption in diesem Bereich.

So weist z.B. das 2-Hydroxy-4-methoxybenzophenon (US-PS 3 751 563) im kurzwelligen UV-A-II-Bereich nur eine verhältnismäßig geringe Absorption auf. Außerdem ist die Löslichkeit der Benzophenone in kosmetischen Ölen begrenzt.

Um einen effektiven Schutzeffekt über den ganzen ultravioletten Bereich zu bekommen, kombiniert man daher häufig verschiedene UV-Filtersubstanzen, die sich in ihrem Absorptionsvermögen ergänzen.

Gesucht werden aber lichtstabile UV-Absorber, die einen breiten Absorptionsbereich aufweisen und die durch eine starke Absorption wirksam vor den Schädigungen durch die UV-A- und UV-B-Strahlen schützen. Für die Verwendung in kosmetischen Sonnenschutzmitteln sollen diese UV-Absorber daneben noch folgende Kriterien erfüllen:
- bei kristallinen UV-Absorbern gute Löslichkeit in kosmetischen Lösungsmitteln und flüssigen, öllöslichen UV-Absorbern, wie z.B. p-Methoxyzimtsäure-ethyl-, -isoamyl- und -isooctylester, Ethylhexylsalicylat, Homomenthylsalicylat, Menthylanthranilat, p-Aminobenzoesäureethylhexylester, 3,3-Diphenyl-2-cyanoacrylsäure-ethyl- und -ethylhexylester;
- flüssige, öllösliche UV-Absorber sollten mit andern UV-Absorbern bzw kosmetischen Ölkomponenten gut mischbar sein;
- wasserresistenter UV-Schutz;
- problemlose Verarbeitbarkeit in kosmetischen Formulierungen und Stabilität unter Anwendungsbedingungen;
- Verträglichkeit mit kosmetischen Grundstoffen;
- pH-Stabilität;
- Thermostabilität;
- keine oder problemlos auswaschbare Verfärbung von Textilien;
- Farblosigkeit und Geruchsneutralität.

Die Erfindung betrifft die Verwendung von Verbindungen der Formel 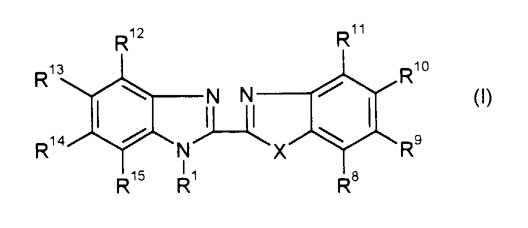 worin bedeuten:
- X: S, NH, NR¹ oder O,
- R¹: C₁-C₂₀-Alkyl, C₂-C₂₀-Alkenyl, C₃-C₁₅-Cycloalkyl, C₆-C₁₂-Aryl, C₆-C₁₂-Ar-C₁-C₆-alkyl, C₁-C₂₀-Alkoxycarbonyl, C₅-C₁₂-Hetaryl, wobei die genannten Substituenten mit C₁-C₆-Alkyl, C₁-C₁₆-Alkoxy, C₆-C₁₂-Aryloxy, Amino, Hydroxy, CONR²R³, COOR⁴, Si(OR⁷)₃ substituiert oder durch Ether-Sauerstoff unterbrochen sein können,
- R², R³: unabhängig voneinander H oder C₁-C₁₆-Alkyl,
- R⁴: H, C₁-C₁₆-Alkyl, C₆-C₁₂-Aryl oder R⁵-O-(CH₂-CH(R⁶)-O-)ₙCH₂-CH(R⁶)-,
- R⁵: C₁-C₄-Alkyl,
- R⁶: H oder Methyl,
- n: Null oder eine ganze Zahl von 1 bis 4,
- R⁷: C₁-C₄-Alkyl und
- R⁸ bis R¹⁵: unabhängig voneinander Wasserstoff Amino, Nitro oder die unter R¹ angegebene Bedeutung besitzen,
als UV-Absorber.

Weiterer Gegenstand der Erfindung sind Verbindungen I,
worin R¹ für C₅-C₂₀-Alkyl, vorzugsweise C₅-C₁₂-Alkyl, insbesondere 2-Ethyl-hexyl steht.

Bevorzugte Verbindungen I sind solche, in denen R¹ gegebenenfalls substituiertes Alkyl bedeutet, wobei insbesondere verzweigte Alkylsubstituenten bevorzugt sind.

Besonders bevorzugte Verbindungen I sind solche, worin R¹ für C₄-C₁₂-Alkyl, Cyclohexyl-C₁-C₆-alkyl, Benzyl oder C₁-C₁₂-Alkoxycarbonyl-C₁-C₃-alkyl steht.

Andere besonders bevorzugte Verbindungen I sind solche, worin X für O, S oder NR¹ und R¹ für C₄-C₁₂-Alkyl, Cyclohexyl-C₁-C₆-alkyl, Benzyl, C₁-C₄-Alkoxycarbonyl-C₁-C₃-alkyl oder C₆-C₁₂-Aryl stehen.

Die Verbindungen der Formel I können analog bekannter Verfahren hergestellt werden, vgl. hierzu z.B. Comprehensive Heterocyclic Chemistry (Editor K.T. Potts, Pergamon Press, 1984), Vol. 5, 387f; Preston, P.N., Chem. Rev. 74, 279 (1974); Demirayak, S., Acta Pharm. Turc. 1991, 33(2), 35; Ennis, B.C., Holan, G. u.L., J. Chem. Soc., C 1967(1), 33. Nachfolgend werden exemplarisch drei mögliche Herstellungsverfahren angegeben:

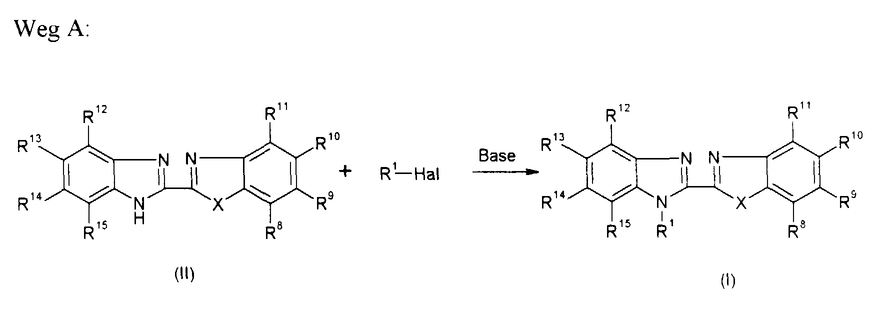

Verbindungen der Formel I können durch Alkylierung von am Stickstoff unsubstituierten Benzimidazolbenzazolen mit Organylhalogeniden erhalten werden.

Die als Edukte verwendeten N-unsubstituierten Benzimidazolbenzazole sind z.B. durch Umsetzung von 2-Trichlormethylbenzimidazol mit o-Phenylendiamin, o-Aminophenol oder o-Aminothiophenol zugänglich. Bisbenzimidazole lassen sich vorteilhafterweise auch durch Erhitzen von o-Phenylendiamin mit Trichloressgsäure in verdünnter Salzsäure darstellen.

Die Organylierung wird unter alkalischen Bedingungen durchgeführt, wobei als Basen Alkali- bzw. Erdalkalihydride, -alkoholate, -hydroxide oder -carbonate verwendet werden können und vorzugsweise pro Mol II 1,0 bis 3,0 mol, insbesondere 1,0 bis 1,5 mol Base verwendet werden. Von dem Organylhalogenid werden in der Regel 1,0 bis 4,0 mol, bevorzugt 1,0 bis 2,0 mol eingesetzt.

Die Organylierung wird bevorzugt in organischen aprotischen Lösungsmitteln, wie z.B. Dimethylformamid oder Dimethylsulfoxid durchgeführt, kann aber auch in Alkoholen, Ethern wie Dioxan, Ketonen wie Aceton, aromatischen Kohlenwasserstoffen wie Toluol oder flüssigem Ammoniak vorgenommen werden. Die Reaktionstemperatur kann im Bereich zwischen 20 und 200°C, insbesondere zwischen 60 und 140°C liegen.

Nach dem Reaktionsende kann durch Zugabe von Wasser das Produkt ausgefällt und durch Umkristallisation bzw. Säulenchromatographie gereinigt werden.

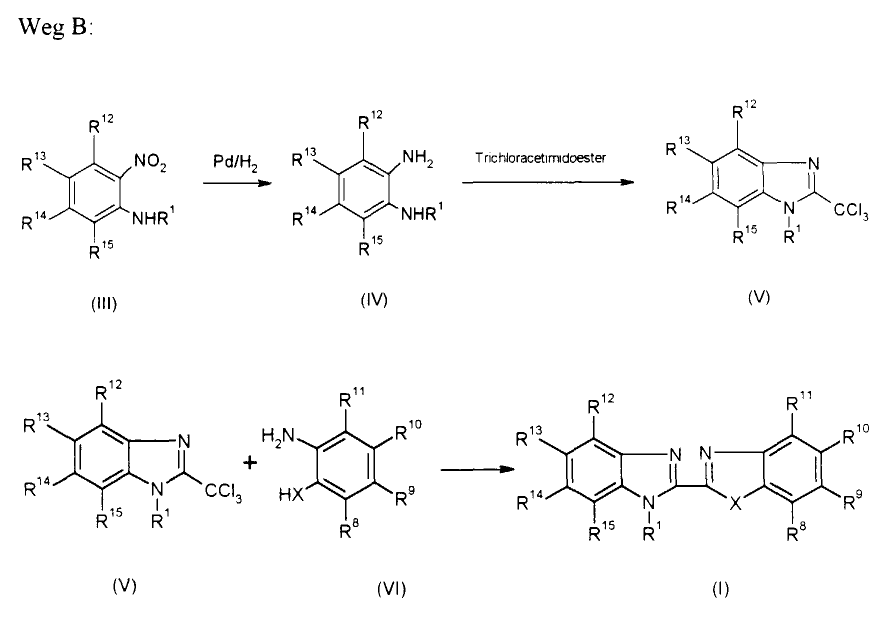

Ans den jeweiligen Nitroverbindungen III sind durch Hydrierung mittels Pd/C N-substituierte o-Phenylendiamine IV zugänglich. Diese lassen sich mit einer äquimolaren Menge Trichloracetimidoester in Essigsäure in die entsprechenden 2-Trichlormethylbenzimidazole V überführen. Die Reaktion verläuft bei Raumtemperatur in 5 bis 20 Stunden. Die Produkte können durch Wasserzugabe ausgefällt werden.

Durch Umsetzung von 1 mol 2-Trichlormethylbenzimidazol V mit 1,0 bis 1,5 mol o-Phenylendiamin, o-Aminophenol oder o-Aminothiophenol VI werden Verbindungen der Formel I erhalten. Die Umsetzung kann bei Temperaturen zwischen 5 und 120°C, bevorzugt 20 bis 80°C, in Alkoholen als Lösungsmittel vorgenommen werden; die Reaktionszeit kann 10 bis 30, bevorzugt etwa 15-20 Stunden betragen.

Um die freiwerdende Salzsäure zu binden, wird die Reaktion vorzugsweise unter Hinzufügen von 3 bis 4 mol Base, vorteilhafterweise eines tertiären Amins, wie z.B. Triethylamin, durchgeführt.

An Stelle von 2-Trichlormethylbenzimidazol können auch Benzimidazol-2-carbonsäurehalogenide, -ester, -amide, -nitrile oder die freie Säure verwendet werden.

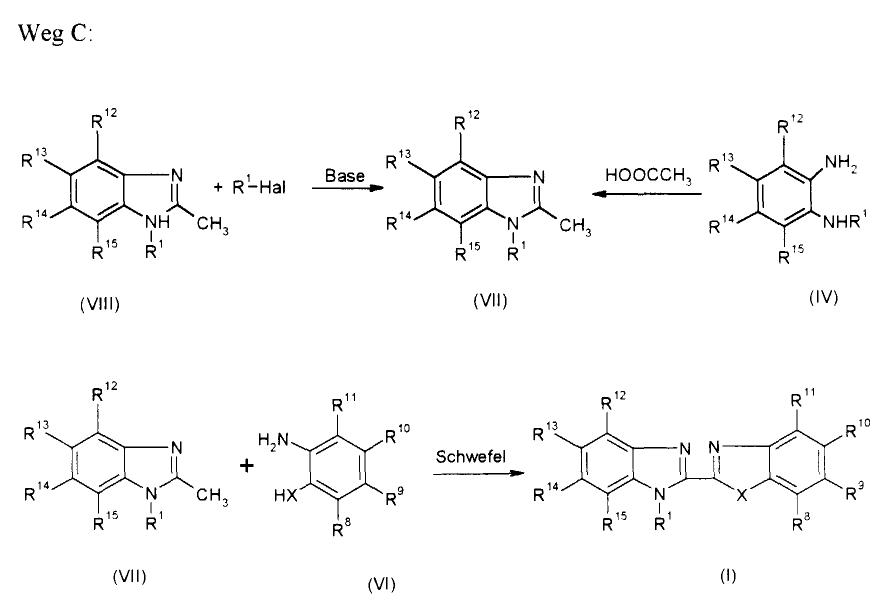

Aus 2-Methylbenzimidazol VIII und Organylhalogeniden können unter alkalischen Bedingungen, wobei als Basen Alkali- bzw. Erdalkalihydride,- alkoholate, -hydroxide oder -carbonate verwendet werden können, die jeweiligen N-substituierten 2-Methylbenzimidazole VII dargestellt werden. In einer bevorzugten Darstellungsform wird die Organylierung in einem 2-Phasensystem, das beispielsweise aus Natronlauge und einem nicht mit Wasser mischbaren organischen Lösungsmittel wie Cyclohexan, Toluol oder Xylol besteht, vorgenommen. Die Umsetzung kann unter Verwendung eines Phasentransferkatalysators, wie z.B. quartären Ammoniumverbindungen, bei Temperaturen von 20 bis 90°C durchgeführt werden. Die Verbindungen VII können auch aus den N-substituierten o-Phenylendiaminen, analog M.A.Philips, J. Chem. Soc. 1928, 2393, durch 5- bis 12-stündiges Erhitzen mit Essigsäure oder Essigsäurederivaten in 4n Salzsäure dargestellt werden.

Die N-substituierten 2-Methylbenzimidazole VII können durch Erhitzen mit äquimolaren Mengen o-Phenylendiamin, o-Aminophenol oder o-Aminothiophenol unter Zusatz von 2,5 bis 5 mol, bevorzugt 3 mol, Schwefel in die Verbindungen der Formel I überführt werden. In einer bevorzugten Ausführungsform wird die Umsetzung durch 8- bis 30-stündiges Erhitzen in Pyridin vorgenommen. Sie kann aber auch durch 8- bis 15-stündiges Erhitzen der Ausgangskomponenten auf 180 bis 250°C sowohl ohne Lösungsmittel als auch unter Verwendung eines inerten, hochsiedenden Lösungsmittels, wie z.B. N-Methylpyrrolidon, 1,2,3,4-Tetrahydronaphthalin oder Diethylenglycolmonoethylether, durchgeführt werden. Nach Abschluß der Reaktion wird das Lösungsmittel abdestilliert oder das Produkt durch Hinzufügen von Wasser ausgefällt. Die Reinigung des Produkts kann durch Säulenchromatographie oder durch Umkristallisation erfolgen.

In analoger Weise ergeben N-substituierte o-Phenylendiamine IV und 2-Methylbenzazole beim Erhitzen mit Schwefel die erfindungsgemäßen Verbindungen I.

Die Darstellung der N-substituierten Benzimidazol-2-yl-benzthiazole gelingt auch durch Umsetzung der Verbindungen VII mit 1,0 bis 1,5 mol Anilin und 2,5 bis 5 mol, bevorzugt 3 mol, Schwefel. Hierzu werden die Ausgangskomponenten 8 bis 20 Stunden auf 120 bis 250°C, vorzugsweise etwa 200°C erhitzt. Die Umsetzung kann ohne Lösungsmittel oder in Gegenwart eines inerten, hochsiedenden Lösungsmittels vorgenommen werden.

Weiterer Gegenstand der Erfindung sind also Verfahren zur Herstellung der Verbindungen I
a) durch Umsetzung von Verbindungen II mit R¹-Halogeniden,
b) durch Umsetzung von 2-Trichlormethyl-benzimidazolen V mit o-Phenylendiamin, o-Aminophenol oder Aminothiophenol und
c) durch Umsetzung von 2-Methylbenzimidazol mit o-Phenylendiamin o-Aminophenol oder o-Aminothiophenol in Gegenwart von Schwefel.

Verbindungen der Formel I sind im Prinzip aus der EP-A 669 323 bekannt; allerdings werden dort Verbindungen, die gleichzeitig am Stickstoff substituiert sind und frei von Sulfonsäuregruppen sind, namentlich nicht genannt. Die aus der EP-A 669 323 bekannten Verbindungen sind also nicht Gegenstand der vorliegenden Erfindung.

Aus der DE-OS 27 33 439 sind N-substituierte Benzimidazole und ihre Verwendung als optische Aufheller bekannt. Einen Hinweis auf die Verwendung dieser Verbindungen als UV-Absorber enthält die DE-OS 27 33 439 nicht.

Die Verbindungen I können insbesondere in kosmetischen pharmazeutischen Sonnenschutzmitteln, Produkten zur Haarpflege und Tagespflege mit UV-Schutz, aber auch als Alterungsschutzmittel für technische Produkte verwendet werden.

Sie zeichnen sich durch eine starke Absorption sowohl im UV-B- als auch in den UV-A-I- und -II-Bereichen aus. Außerdem weisen sie eine ausgezeichnete Lichtbeständigkeit und eine gute Löslichkeit in kosmetischen Lösungsmitteln und in flüssigen öllöslichen UV-Absorbern auf.

Die UV-Absorber I eignen sich aufgrund ihres hydrophoben Charakters insbesondere auch für die Formulierung wasserresistenter Sonnenschutzprodukte.

Die Absorber I verhindern als UV-A/B-Breitbandabsorber beim Einsatz in kosmetischen oder pharmazeutischen Zubereitungen den Durchtritt der UV-Strahlen durch den aufgetragenen Film der Zubereitung. Dies ist im allgemeinen der Fall, wenn die Zubereitungen 0,5 bis 15, vorzugsweise 1 bis 10, insbesondere 2 bis 7, Gew.-% (bezogen auf das Gesamtgewicht der Zubereitung) Verbindung I enthalten.

Die Verbindungen I eignen sich auch zur Photostabilisierung von UV-Absorbern mit geringer UV-Lichtstabilität. Insbesondere gelingt die Photostabilisierung der sehr lichtinstabilen Verbindungen der Dibenzoylmethane, insbesondere 4-tert.-Butyl-4'-methoxydibenzoylmethan und 4-Isopropyldibenzoylmethan.

Die Verbindungen I enthaltenden Zubereitungen können zum Schutz der Haut und der Haare - insbesondere durch Dauerwelle, Färbung und Bleichung bereits vorgeschädigter Haare - vor UV-Bestrahlung verwendet werden. Diese zum Schutz von Haut und Haaren vor der UV-Strahlung dienenden kosmetischen und pharmazeutischen Zubereitungen können in den üblicherweise verwendeten Anwendungsformen vorliegen, d.h. als Öl-in-Wasser- oder Wasser-in-Öl-Emulsion, als Milch, als Lotion oder Creme, Aerosol, Hydrodispersions-Gel (emulgatorfrei) oder als jegliche andere übliche kosmetische oder pharmazeutische Zubereitung. Für den Schutz der Haare vor UV-Strahlen werden bevorzugt Zubereitungen als Shampoo, Konditionierungsmittel, Kombinationen (z.B. 2 in 1), Spülung, Kur, Gel, Lotion, Spray oder Creme verwendet.

Die kosmetischen und pharmazeutischen Zubereitungen können die in diesen Mitteln üblicherweise verwendeten Bestandteile, wie z.B. Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester (z.B. Isopropylpalmitat, Isooctylstearat, Adipinsäurediisopropylester usw.), natürliche oder synthetische Öle oder Wachse, Pigmente (z.B. Titandioxid, Zinkoxid, Perglanzpigmente, Farbpigmente), Verdickungsmittel (z.B. Hydroxyethylcellulose, Bentonit usw.), Konservierungsstoffe, Feuchtigkeitsmittel, Vitamine, Siliconöle, Glycerin, Ethylalkohol, Parfumöle, Chelatisierungsmittel, Antioxidantien enthalten.

Die Verbindungen I können einzeln oder in Mischung in den entsprechenden Zubereitungen eingesetzt werden; man kann sie auch in Kombination mit UV-Absorbern anderer Substanzklassen einsetzen. Beispiele solcher Verbindungen umfassen
p-Aminobenzoesäure
p-Aminobenzoesäureethylester (25 mol) ethoxyliert
p-Dimethylaminobenzoesäure-2-ethylhexylester
p-Aminobenzoesäureethylester (2 mol) N-propoxyliert
p-Aminobenzoesäureglycerinester
Salicylsäure-homomenthylester
Salicylsäure-2-ethylhexylester
Triethanolaminsalicylat
4-Isopropylbenzylsalicylat
Anthranilsäurementhylester
Diisopropylzimtsäureethylester
p-Methoxyzimtsäure-2-ethylhexylester
Diisopropylzimtsäuremethylester
p-Methoxyzimtsäureisoamylester
p-Methoxyzimtsäure-diethanolaminsalz
p-Methoxyzimtsäure-isopropylester
2-Ethylhexyl-2-cyano-3,3-diphenylacrylat
Ethyl-2-cyano-3,3'-diphenylacrylat
2-Phenylbenzimidazolsulfonsäure und ihre Salze
3-(4'-Trimethylammonium)-benzyliden-bornan-2-on-methylsulfat
Terephtalyliden-dibornansulfonsäure und ihre Salze
4-tert.-Butyl-4'-methoxy-dibenzoylmethan
β-Imidazol-4(5)-acrylsäure (Urocaninsäure)
2-Hydroxy-4-methoxybenzophenon
2-Hydroxy-4-methoxybenzophenon-5-sulfonsäure
Dihydroxy-4-methoxybenzophenon
2,4-Dihydroxybenzophenon
Tetrahydroxybenzophenon
2,2'-Dihydroxy-4,4'-dimethoxybenzophenon
2-Hydroxy-4-n-octoxybenzophenon
2-Hydroxy-4-methoxy-4'-methylbenzophenon
3-(4'-Sulfo)benzyliden-bornan-2-on und dessen Salze
3-(4'-Methylbenzyliden)-d,1-campher
4-Isopropyldibenzoylmethan
2,4,6-Trianilino-(p-carbo-2'-ethylhexyl-1'-oxy)-1,3,5-triazin
Phenylen-bis-benzimidazyl-tetrasulfonsäure und ihre Salze
N-(2 und 4)-[(2-oxoborn-3-yliden)methyl]benzyl]-acrylamid-Polymer
Polysiloxanmalonate.

Besonders geeignete UV-Absorber sind:
p-Methoxyzimtsäure-2-ethylhexylester,
p-Methoxyzimtsäure-isoamylester,
2-Phenylbenzimidazolsulfonsäure,
3-(4'-Methylbenzyliden)-d,1-campher,
2-Ethylhexyl-2-cyano-3,3-diphenylacrylat,
Salicylsäure-2-ethylhexylester,
4-tert.-Butyl-4'-methoxydibenzoylmethan,
Phenylen-bis-benzimidazyl-tetrasulfonsäure und ihre Salze
Anthranilsäurementhylester sowie
2,4,6-Trianilino-(p-carbo-2'-ethylhexyl-1'-oxy)-1,3,5-triazin.

Ebenso ist die Kombination der Verbindungen I mit feinteiligen (beschichteten oder unbeschichteten) Pigmenten, wie z.B. Titandioxid, Zinkoxid und Eisenoxid, in Sonnenschutz- und Tagespflegeprodukten mit UV-Schutz möglich.

Weiterhin lassen sich die erfindungsgemäßen Verbindungen I auch mit UV-Absorbern, die für den technischen Produktschutz eingesetzt werden, kombinieren.

Beispiele solcher UV-Absorber entsprechen vorwiegend Verbindungen aus der Reihe der Benzotriazole, Benzophenone und Malonsäureester.

Die Prozentangaben der nachfolgenden Beispiele beziehen sich jeweils auf das Gewicht. Soweit nicht anders angegeben, erfolgte die Trennung oder Reinigung der Verbindungen per Säulenchromatographie an Kieselsol 60 (Elution mit n-Hexan/Essigsäureethylester = (5-10):1).

### Beispiele

### Beispiel 1-13

### Allgemeine Vorschrift zur Herstellung von Benzimidazol-2-yl-benzoxazolen

### (Weg A)

7,05g (0,03 mol) 2-(Benzimidazol-2-yl)-benzoxazol werden in 50 ml Dimethylformamid vorgelegt, 0,72 g (0,03 mol) Natriumhydrid oder 4,8 g (0,03 mol) 50 %-ige Natronlauge zugegeben und 60 Minuten auf 90°C erhitzt. Danach werden 0,045 mol des entsprechenden Alkylbromids bzw. -chlorids, für die Verbindungen in den Beispielen 10 und 11 4,32g (0,06 mol) 1,2-Epoxybutan, zugefügt und 8 Stunden bei 120°C gerührt. Nach Abkühlen auf Raumtemperatur wird das Rohprodukt durch Zugabe von Wasser ausgefällt und durch Umkristallisation oder Säulenchromatographie gereinigt.

### Beispiel 14

### 2-(1-(2-Ethylhexyl)benzimidazol-2-yl)-benzoxazol (Weg C)

24.4g (0,1mol) 1-(2-Ethylhexyl)-2-methylbenzimidazol (hergestellt durch Erhitzen von N-2-Ethylhexyl-o-phenylendiamin in Essigsäure/Salzsäure), 10,9 g (0,1 mol) o-Aminophenol und 9,6 g (0,3 mol) Schwefel werden in 100 ml Pyridin 18 Stunden unter Rückfluß erhitzt. Nach Abkühlen auf Raumtemperatur wird das Rohprodukt durch Zugabe von Wasser ausgefällt. Nach mehrfachem Umkristallisieren aus n-Hexan unter Zusatz von Aktivkohle werden 5,7 g (16 % der Theorie) Produkt erhalten, das nach Schmelzpunkt, HPLC- und UV-Bestimmung mit der im Beispiel 7 erhaltenen Verbindung identisch ist.

### Beispiel 15

### 2-(1-(2-Ethylhexyl)-benzimidazol-2-yl)-benzoxazol (Weg B)

28,2 g (0,12 mol) 1-(2-Ethylhexyl)-2-trichlormethylbenzimidazol (hergestellt durch Umsetzung von N-2-Ethylhexyl-o-phenylendiamin mit Trichloracetimidomethylester), 13,1 g (0,12 mol) o-Aminophenol und 39,4 g (0,38 mol) Triethylamin werden in 150 ml Ethanol 15 Stunden bei Raumtemperatur gerührt. Anschließend wird das Ethanol am Rotationsverdampfer entfernt, der verbleibende Rückstand mit Essigester versetzt, mit Wasser gewaschen und mit Natriumsulfat getrocknet. In dem so erhaltenen Produktgemisch werden durch HPLC-Bestimmung 35 % Produkt identifiziert, die Isolierung kann beispielsweise durch Säulenchromatographie erfolgen.

### Beispiel 16-18

### Allgemeine Vorschrift zur Herstellung von Benzimidazol-2-yl-benzthiazolen

7,53 g (0,03 mol) 2-(Benzimidazol-2-yl)-benzthiazol werden in 50 ml Dimethylformamid vorgelegt, 0,72 g (0,03 mol) Natriumhydrid oder 4,8 g (0,03 mol) 50 %ige Natronlauge zugegeben und 60 Minuten auf 90°C erhitzt. Danach werden 0,045 mol des entsprechenden Alkylbromids bzw. -chlorids zugeführt und 8 Stunden bei 120°C gerührt. Nach Abkühlen auf Raumtemperatur wird das Rohprodukt durch Zugabe von Wasser ausgefällt und durch Umkristallisation oder Säulenchromatographie gereinigt.

### Beispiel 19

### 2-(1-(2-Ethylhexyl)benzimidazol-2-yl)-benzthiazol (Weg C)

48,8 g (0,2 mol) 1-(2-Ethylhexyl)-2-methylbenzimidazol (hergestellt durch Alkylierung von 2-Methylbenzimidazol mit 2-Ethylhexylbromid unter Phasentransferbedingungen), 25 g (0,2 mol) o-Aminothiophenol und 19,2 g (0,6 mol) Schwefel in 200 ml Pyridin werden 18 Stunden unter Rückfluß erhitzt. Anschließend wird das Pyridin abdestilliert und der verbleibende Rückstand durch Erwärmen in 80 g Ethanol gelöst.

Nach Abkühlen scheiden sich 55,2 g (76 % der Theorie) Produkt ab, das nach Schmelzpunkt, HPLC- und UV-Bestimmungen mit der nach Weg A im Beispiel 18 erhaltenen Verbindung identisch ist.

### Beispiel 20

### 2-(1-(2-Ethylhexyl)benzimidazol-2-yl)-benzthiazol (Weg C)

24,4 g (0,1 mol) 1-(2-Ethylhexyl)-2-methylbenzimidazol 9,3 g (0,1 mol) Anilin und 9,6 g (0,3 mol) Schwefel werden 18 Stunden auf 200°C erhitzt. Nach Abkühlen auf 60°C werden 10 ml Ethanol hinzugefügt und von unlöslichen Bestandteilen abfiltriert. In dem so erhaltenen Rohprodukt werden durch gaschromatographische Bestimmung 54,4 % Produkt identifiziert; die Isolierung kann beispielsweise durch Säulenchromatographie erfolgen.

### Beispiel 21-27

### Allgemeine Vorschrift zur Herstellung von 2,2'-Bisbenzimidazolen

7,02 g (0,03 mol) 2,2'-Bisbenzimidazol werden in 50 ml Dimethylformamid vorgelegt, 1,44 g (0,06 mol) Natriumhydrid oder 9,6 g (0,06 mol) 50 %ige Natronlauge zugegeben und 60 Minuten auf 90°C erhitzt. Danach werden 0,09 mol des entsprechenden Alkylbromids bzw. -chlorids zugefügt und 8 Stunden bei 120°C gerührt Nach Abkühlen auf Raumtemperatur wird das Rohprodukt durch Zugabe von Wasser ausgefällt und durch Umkristallisation oder Säulenchromatographie gereinigt.

### Beispiel 28 und 29

### 1,1'-Dipentyl-5-methoxy- und 1,1'-Dipentyl-6-methoxy-2,2'-bisbenzimidazol

7,9 g (0,03 mol) 5-Methoxy-2,2'-bisbenzimidazol werden in 50 ml Dimethylformamid vorgelegt, 1,73 g (0,072 mol) Natriumhydrid zugegeben und 60 Minuten auf 90°C erhitzt. Danach werden 18,1 g (0,12 mol) n-Pentylbromid zugefügt und 8 Stunden bei 120°C gerührt. Nach Abkühlen auf Raumtemperatur wird durch Zugabe von Wasser das Rohprodukt ausgefällt. Dieses besteht aus einem Gemisch der isomeren 1,1'-Dipentyl-5-methoxy-(26) und 1,1'-Dipentyl-6-methoxy-2,2'-bisbenzimidazole (27), die durch Säulenchromatographie an Chiracel OG (Methanol/Wasser = 9:1) getrennt werden.

### Beispiel 30

### 1,1'-Dipropyl-5,5'-dimethyl- und 1,1'-Dipropyl-6,6'-dimethyl-2,2'-bisbenzimidazol

4,1 g (0,053 mol) 5,5'-Dimethyl-2,2'-bisbenzimidazol, 5,1 g (0,127 mol) Natriumhydrid und 36 g (0,21 mol) n-Propyljodid werden analog der allgemeinen Vorschrift für die Herstellung von 2,2'-Bisbenzimidazole umgesetzt. Das erhaltene Rohprodukt besteht aus einem Gemisch aus 1,1'-Dipropyl-5,5'-dimethyl- und 1,1'-Dipropyl-6,6'-dimethyl-2,2'-bisbenzimidazol, das durch Säulenchromatographie gereinigt wird.

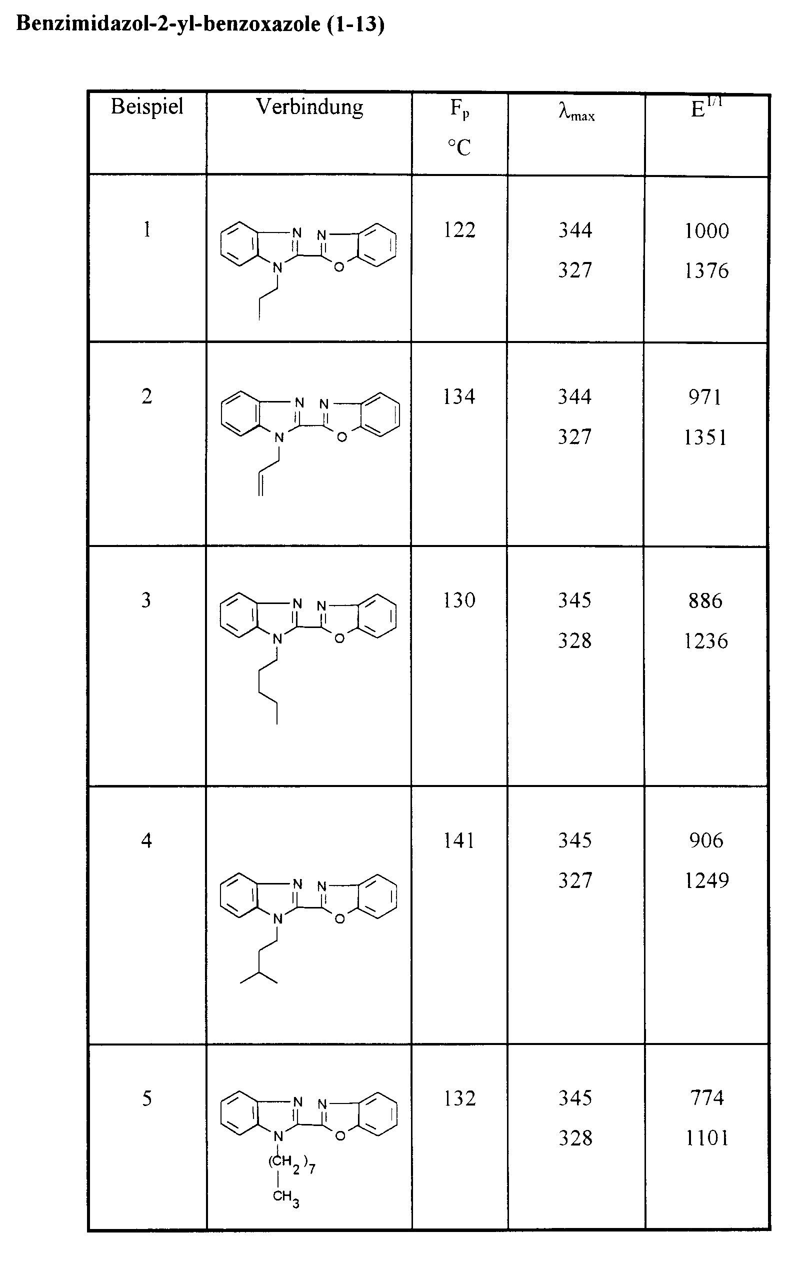

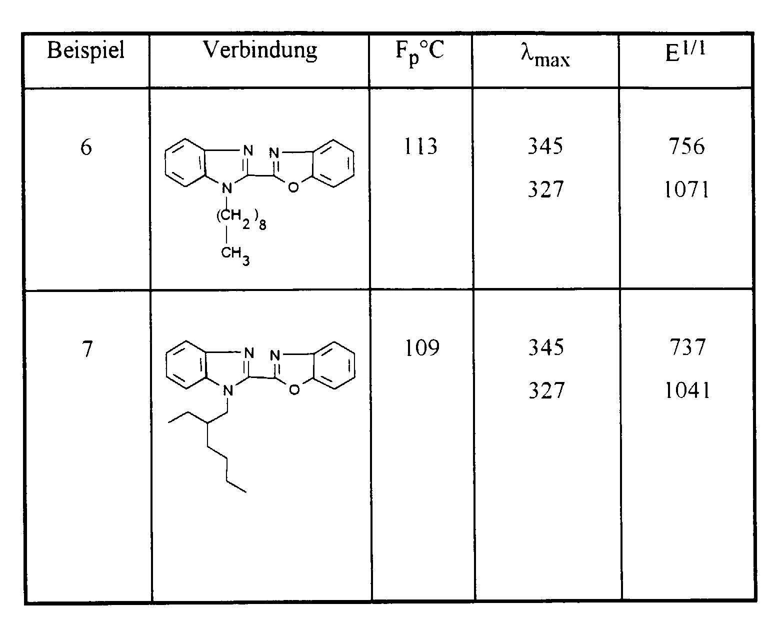

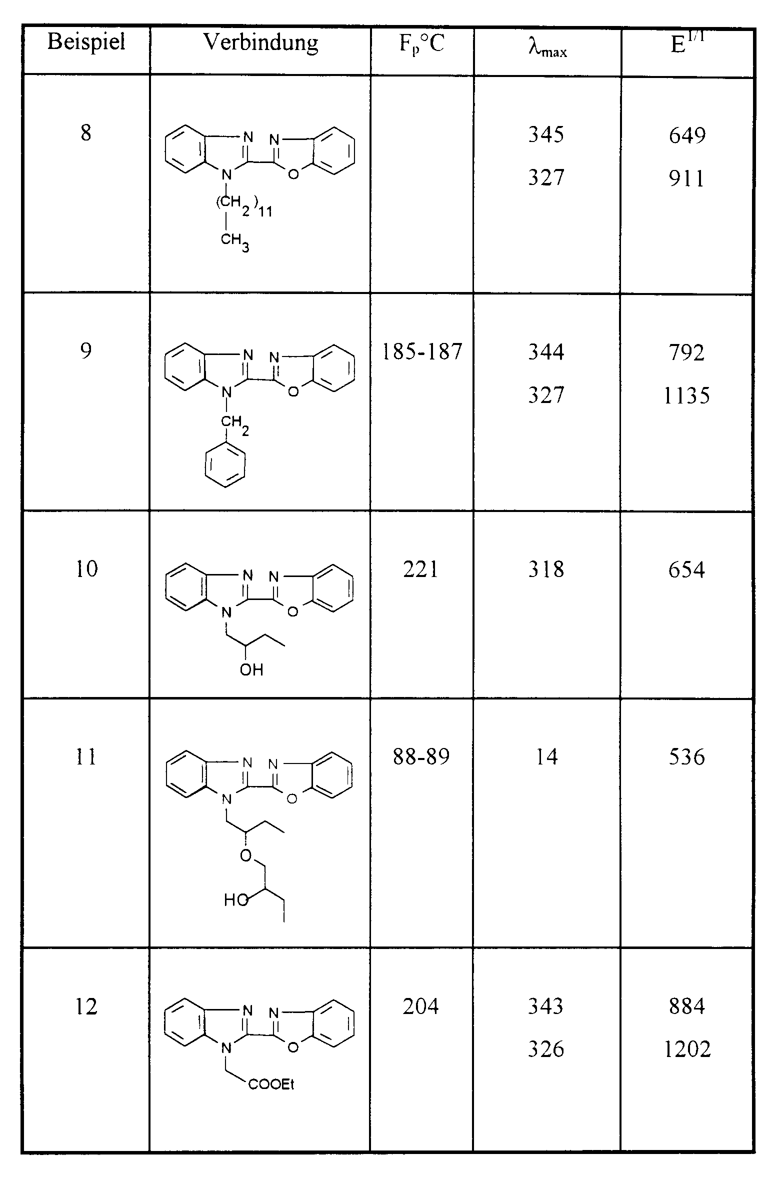

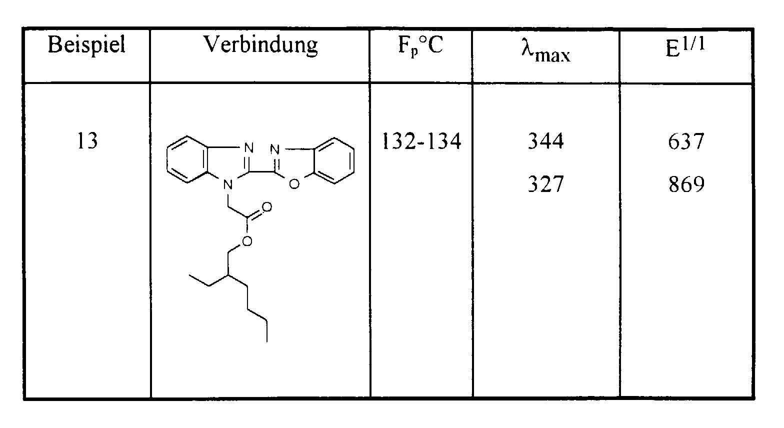

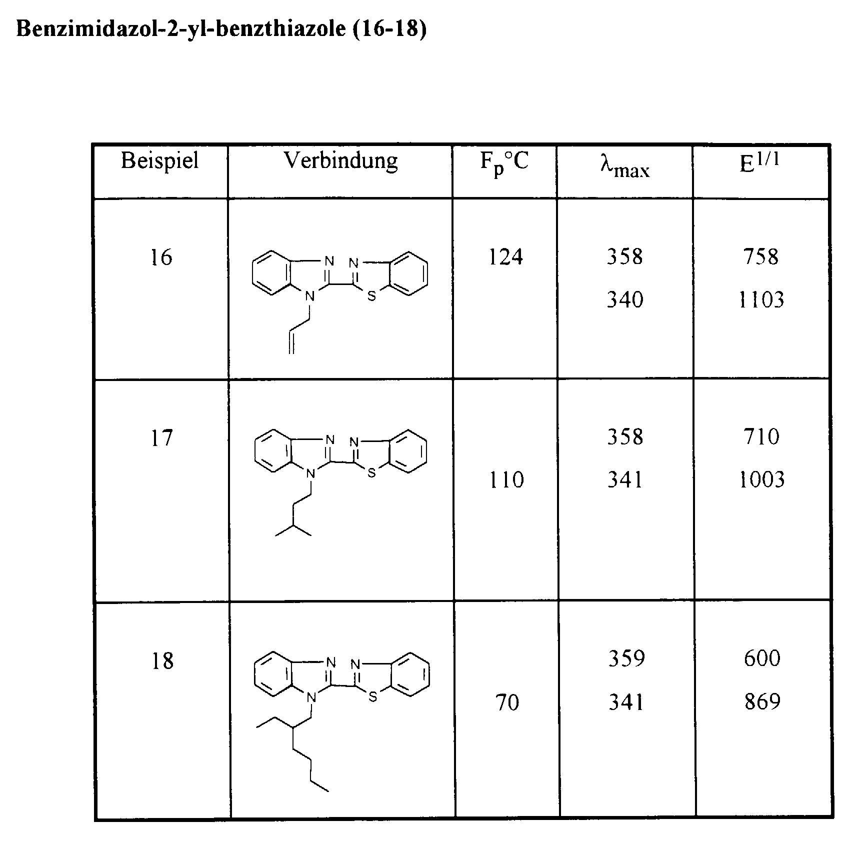

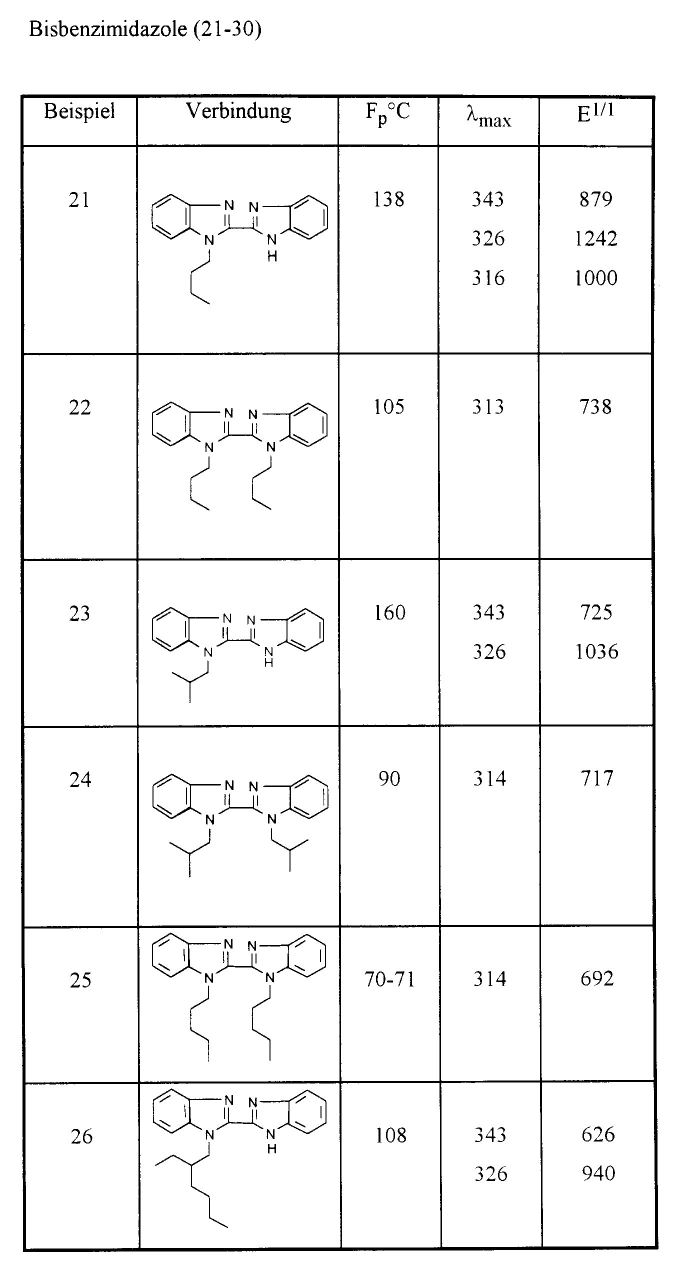

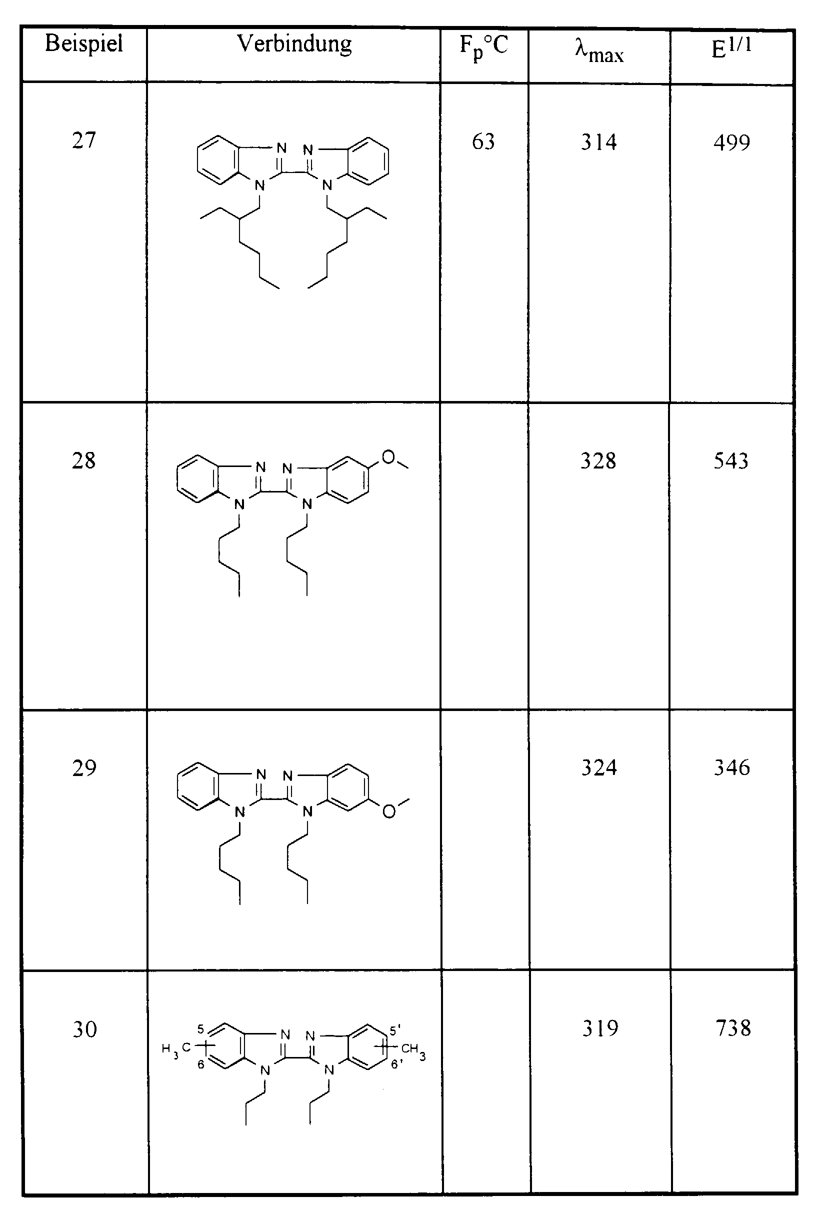

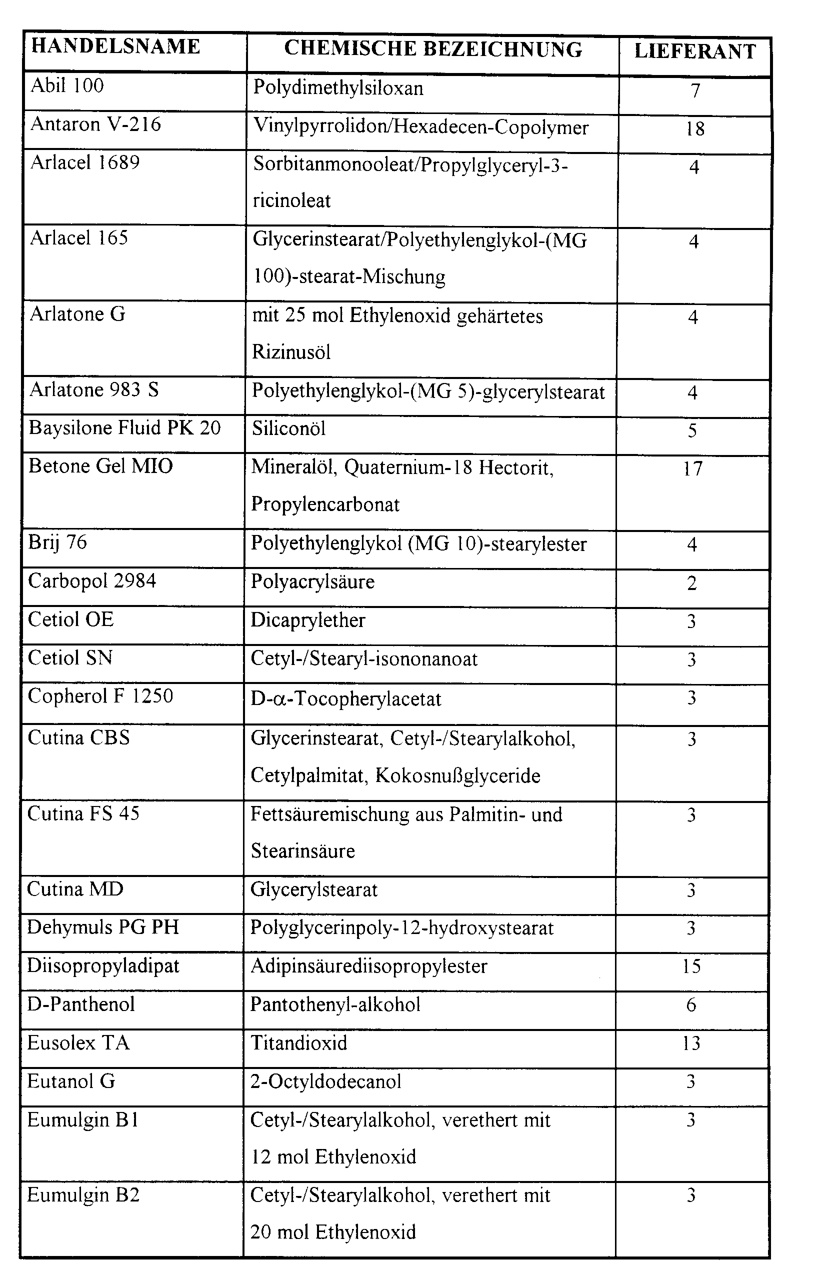

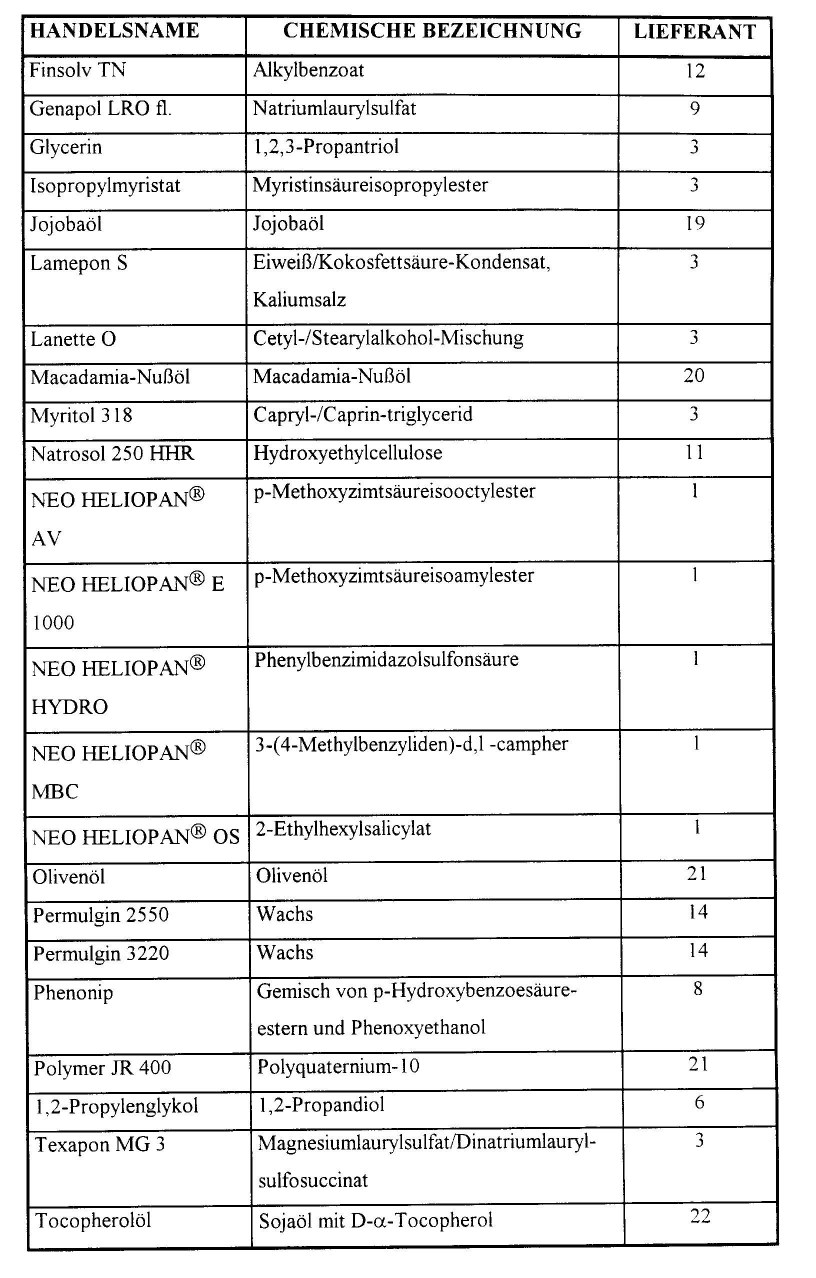

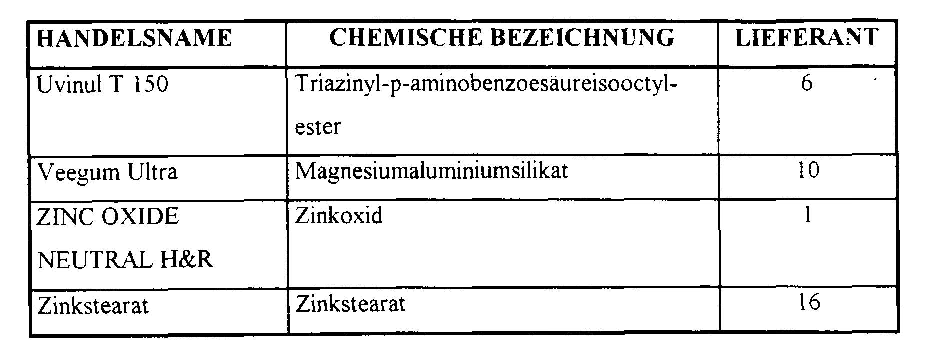

| **LIEFERANTEN** | | |
|---|---|---|
| | | |
| 1. | Haarmann & Reimer GmbH, Holzminden | |
| 2. | B.F. Goodrich Company, Neuss | |
| 3. | Henkel KGaA, Düsseldorf | |
| 4. | ICI Speciality Chemicals, Frankfurt | |
| 5. | Bayer AG, Leverkusen | |
| 6. | BASF, Ludwigshafen | |
| 7. | Goldschmidt AG, Essen | |
| 8. | Nipa Lab. Ltd., Pontypridd Mid Glam, Wales / GB | |
| 9. | Hoechst AG, Frankfurt | |
| 10. | R.T. Vanderbilt Company Inc., Norwalk / USA | |
| 11. | Hercules Inc., Wilmington, Delaware / USA | |
| 12. | Witco Surfactants GmbH, Steinau a.d. Straße | |
| 13. | E. Merck, Darmstadt | |
| 14. | Koster Keunen Holland BV, Bladl / NL | |
| 15. | Akzo Chemie GmbH, Düren | |
| 16. | Chemische Werke Bärlocher, München | |
| 17. | Rheox Inc., Hightstown, New Jersey / USA | |
| 18. | ISP Global Technologies Deutschland GmbH, Frechen | |
| 19. | Henry Lamotte, Bremen | |
| 20. | Erhard Wagner GmbH, Bremen | |
| 21. | Nordmann & Rassmann GmbH & Co., Hamburg | |
| 22. | Richter GmbH, Berlin | |
| | | |
| | | |

### Beispiel 31

| **SONNENSCHUTZLOTION (O/W)** | | |
|---|---|---|
| | BESTANDTEILE | % |
| | | |
| A) | Arlatone 983 S | 1,75 |
| | Brij 76 | 1,25 |
| | Lanette 0 | 1,15 |
| | Myritol 318 | 5,00 |
| | Eutanol G | 30,00 |
| | Cetiol SN | 5,00 |
| | UV-Absorber gemäß Formel (I) | 2,00 |
| | | |
| B) | Wasser, dest. | 24,65 |
| | 1,2-Propylenglykol | 2,00 |
| | Phenonip | 0,50 |
| | | |
| C) | Wasser, dest. | 25,00 |
| | Carbopol 2984 | 0,30 |
| | Natriumhydroxid, 10 %ig in Wasser | 1,00 |
| | | |
| D) | Parfumöl | 0,40 |
| | | |

| HERSTELLVORSCHRIFT: | | |
|---|---|---|
| Teil A: | Bei ca. 80°C aufschmelzen. | |
| Teil B: | Auf ca. 90°C erhitzen. Teil B unter Rühren zu Teil A geben. | |
| Teil C: | Carbopol klumpenfrei in Wasser dispergieren, mit Natriumhydroxid-Lösung zu einem Gel neutralisieren, bei ca. 60°C zu Teil A/B geben. Auf Raumtemperatur abrühren. | |
| Teil D: | Bei ca. 30°C die Emulsion parfümieren. pH-Wert kontrollieren (6,5 bis 7,0). | |

### Beispiel 32

| **SONNENSCHUTZMILCH (W/O)** | | |
|---|---|---|
| | BESTANDTEILE | % |
| | | |
| A) | Dehymuls PG PH | 5,00 |
| | Permulgin 3220 | 0,50 |
| | Zinksstearat | 0,50 |
| | Finsolv TN | 19,00 |
| | Eutanol G | 19,00 |
| | UV-Absorber gemäß Formel (I) | 2,00 |
| | | |
| | | |
| B) | Wasser, dest. | 47,50 |
| | Glycerin 86 % | 5,00 |
| | Magnesiumsulfat 7 H₂O | 0,50 |
| | Phenonip | 0,50 |
| | | |
| C) | Parfumöl | 0,50 |
| | | |
| | | |
| | | |
| | | |
| | | |

| HERSTELLVORSCHRIFT: | | |
|---|---|---|
| Teil A: | Bei ca. 90°C aufschmelzen. | |
| Teil B: | Auf ca. 95°C erhitzen, dann Teil B unter Rühren zu Teil A geben. Auf Raumtemperatur abrühren. | |
| Teil C: | Bei 30°C Teil C zugeben und anschließend homogenisieren. | |

### Beispiel 33

| **SONNENSCHUTZMILCH (O/W)** | | |
|---|---|---|
| | BESTANDTEILE | % |
| | | |
| A) | Arlacel 165 | 3,00 |
| | Eumulgin B 2 | 1,00 |
| | Lanette 0 | 1,00 |
| | Myritol 318 | 3,00 |
| | Cetiol OE | 2,00 |
| | Abil 100 | 1,00 |
| | Bentone Gel M10 | 3,00 |
| | Cutina CBS | 1,00 |
| | Phenonip | 0,20 |
| | NEO HELIOPAN® (Octyl Salicylate) | 3,00 |
| | NEO HELIOPAN® AV (Octyl Methoxycinnamate) | 5,00 |
| | NEO HELIOPAN® E 1000 (Isoamyl p-Methoxycinnamate | 5,00 |
| | NEO HELIOPAN® MBC (4-Methylbenzylidene Camphor) | 1,00 |
| | Eusolex TA | 3,00 |
| | UV-Absorber gemäß Formel (I) | 2,00 |
| | Eutanol G | 30,00 |
| | | |
| B) | Wasser, dest. | 17,60 |
| | Glycerin, 86 %ig | 3,00 |
| | Phenonip | 0,30 |
| | Veegum Ultra | 1,00 |
| | Natrosol 250 HHR | 0,30 |
| | NEO HELIOPAN® HYDRO, eingesetzt als 15 %ige Lösung nach Neutralisation mit Natriumhydroxid (Phenyl benzimidazolsulfonsäure) entspricht Aktivsubstanz: 2,0 % | 13,30 |
| | | |
| C) | Parfumöl | 0,30 |

| HERSTELLVORSCHRIFT | | |
|---|---|---|
| Teil A: | Bei ca. 80°C aufschmelzen, dann Eusolex TA sorgfältig dispergieren. | |
| Teil B: | Ohne Veegum und Natrosol auf ca. 90°C erhitzen, dann Veegum und Natrosol dispergieren. Teil B unter Rühren zu Teil A geben. Auf Raumtemperatur abrühren. | |
| Teil C: | Bei 30°C Teil C zugeben und anschließend homogenisieren. pH-Wert überprüfen (7,0 - 7,5). | |

### Beispiel 34

| **SONNENSCHUTZLOTION (W/O)** | | |
|---|---|---|
| | BESTANDTEILE | % |
| | | |
| A) | Arlacel 1689 | 3,50 |
| | Finsolv TN | 6,00 |
| | NEO HELIOPAN® E 1000 (Isoamyl-p-methoxycinnamat) | 7,00 |
| | Uvinul T 150 (Octyl-triazon) | 1,00 |
| | UV-Absorber gemäß Formel (I) | 2,00 |
| | Copherol F 1250 | 2,00 |
| | Permulgin 2550 | 1,00 |
| | Myritol 318 | 6,00 |
| | Eutanol G | 28,50 |
| | ZINKOXID NEUTRAL H&R (Zinc Oxide) | 7,00 |
| | | |
| B) | Wasser, dest. | 30,20 |
| | Glycerin 86 % | 5,00 |
| | Phenonip | 0,50 |
| | | |
| C) | Parfumöl | 0,30 |
| | | |

| HERSTELLVORSCHRIFT: | | |
|---|---|---|
| Teil A: | Bei ca. 90°C sorgfältig aufschmelzen (ohne ZINKOXID NEUTRAL H&R). Anschließend ZINKOXID NEUTRAL H&R sorgfältig dispergieren. | |
| Teil B: | Auf ca. 95°C erhitzen, dann Teil B unter Rühren zu Teil A geben. Auf Raumtemperatur abrühren. | |
| Teil C: | Bei 30°C Teil C zugeben und anschließend homogenisieren. | |

### Beispiel 35

| **SONNENSCHUTZÖL** | | |
|---|---|---|
| | BESTANDTEILE | % |
| | | |
| A) | NEO HELIOPAN® E 1000 (Isoamyl-p-methoxycinnamat) | 7,50 |
| | NEO HELIOPAN® OS (Octylsalicylat) | 5,00 |
| | UV-Absorber gemäß Formel (I) | 3,00 |
| | Myritol 318 | 34,70 |
| | Diisopropyladipat | 5,00 |
| | Olivenöl | 1,00 |
| | Jojobaöl | 1,00 |
| | Macadamia Nußöl | 1,00 |
| | Tocopherolöl | 1,00 |
| | Isopropylmyristat | 35,00 |
| | Antaron V-216 | 5,00 |
| | Phenonip | 0,50 |
| | Parfumöl | 0,30 |
| | | |
| | | |
| | | |
| | | |
| | | |
| | | |

| HERSTELLVORSCHRIFT: | | |
|---|---|---|
| Alle Bestandteile sorgfältig vermischen, gegebenenfalls leicht erwärmen, bis alle Bestandteile gelöst sind. | | |
| | | |

### Beispiel 36

| **SONNENSCHUTZLOTION (O/W)** | | |
|---|---|---|
| | BESTANDTEILE | % |
| | | |
| A) | Cutina FS 45 | 2,00 |
| | Eumulgin B1 | 0,25 |
| | Eumulgin B2 | 0,25 |
| | Cutina MD | 2,00 |
| | Lanette O | 2,80 |
| | Eutanol G | 19,00 |
| | Finsolv TN | 19,00 |
| | UV-Absorber gemäß Formel (I) | 2,00 |
| | | |
| B) | Wasser, dest. | 24,90 |
| | Phenonip | 0,50 |
| | | |
| C) | Wasser, dest. | 25,00 |
| | Carbopol 2984 | 0,40 |
| | Natriumhydroxid, 10 %ig in Wasser | 1,50 |
| | | |
| D) | Parfumöl | 0,40 |
| | | |

| HERSTELLVORSCHRIFT: | | |
|---|---|---|
| Teil A: | Bei ca. 80°C aufschmelzen. | |
| Teil B: | Auf ca. 90°C erhitzen. Teil B unter Rühren zu Teil A geben. | |
| Teil C: | Carbopol klumpenfrei in Wasser dispergieren, mit Natriumhydroxid-Lösung zu einem Gel neutralisieren, bei ca. 60°C zu Teil A/B geben. Auf Raumtemperatur abrühren. | |
| Teil D: | Bei ca. 30°C die Emulsion parfümieren. pH-Wert kontrollieren (6,5 bis 7,0). | |

### Beispiel 37

| **HAARSHAMPOO** | | |
|---|---|---|
| | BESTANDTEILE | % |
| | | |
| A) | Genapol LRO flüssig | 18,00 |
| | Texapon MG3 | 36,00 |
| | Lamepon S | 6,00 |
| | Parfumöl | 0,60 |
| | Phenonip | 0,50 |
| | Arlatone G | 2,00 |
| | UV-Absorber gemäß Formel (I) | 0,20 |
| | NEO HELIOPAN® E 1000 (Isoamyl-p-methoxycinnamat) | 1,00 |
| | | |
| B) | Wasser, dest. | 33,30 |
| | Polymer JR 400 | 0,20 |
| | D-Panthenol | 1,00 |
| | Natriumchlorid | 1,00 |
| | Natriumhydroxid, 10 %ig in Wasser | 0,20 |
| | | |
| | | |

| HERSTELLVORSCHRIFT: | | |
|---|---|---|
| Teil A: | UV-Absorber in NEO HELIOPAN® E 1000 und Phenonip unter schwachem Erwärmen lösen, dann Arlatone G und Parfumöl zugeben und gut vermischen. Restliche Bestandteile einwiegen. | |
| Teil B: | Polymer unter Rühren im Wasser lösen, restliche Bestandteile zugeben und lösen. Teil B zu Teil A geben verrühren (pH-Wert kontrollieren, ca. 5,5). | |

## Patentansprüche

1. Verwendung von Verbindungen der Formel 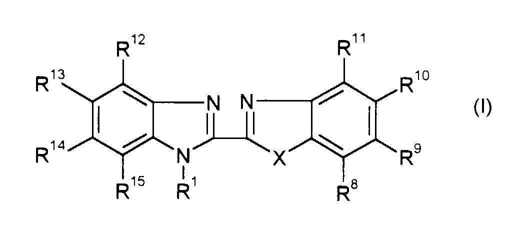 worin bedeuten:
X S, NH, NR¹ oder O,
R¹ C₁-C₂₀-Alkyl, C₂-C₂₀-Alkenyl, C₃-C₁₅-Cycloalkyl, C₆-C₁₂-Aryl, C₆-C₁₂-Ar-C₁-C₆-alkyl, C₁-C₂₀-Alkoxycarbonyl, C₅-C₁₂-Hetaryl, wobei die genannten Substituenten mit C₁-C₆-Alkyl, C₁-C₁₆-Alkoxy, C₆-C₁₂-Aryloxy, Amino, Hydroxy, CONR²R³, COOR⁴, Si(OR⁷)₃ substituiert sein können,
R², R³ unabhängig voneinander H oder C₁-C₁₆-Alkyl,
R⁴ H, C₁-C₁₆-Alkyl, C₆-C₁₂-Aryl oder R⁵ -O-(CH₂-CH(R⁶)-O-)n-CH₂-CH(R⁶)-,
R⁵ C₁-C₄-Alkyl,
R⁶ H oder Methyl,
n Null oder eine ganze Zahl von 1 bis 4,
R⁷ C₁-C₄-Alkyl und
R⁸ bis R¹⁵ unabhängig voneinander Wasserstoff Amino, Nitro oder die unter
R¹ angegebene Bedeutung besitzen,
als UV-Absorber.

2. Verwendung nach Anspruch 1, wobei
X für Sauerstoff oder Schwefel und
R¹ für C₄-C₁₂-Alkyl, Cyclohexyl-C₁-C₆-alkyl, Benzyl oder C₁-C₄-Alkoxycarbonyl-C₁-C₃-alkyl
stehen.

3. Verwendung nach Anspruch 1, wobei
X für NR¹ und
R¹ für C₄-C₁₂-Alkyl, Cyclohexyl-C₁-C₆-alkyl, Benzyl, C₁-C₄-Alkoxycarbonyl-C₁-C₃-alkyl oder C₆-C₁₂-Aryl
stehen.

4. Verbindungen der Formel (I) nach Ansprüchen 1 bis 3, worin R¹ für C₅-C₂₀-Alkyl steht.

5. Verfahren zur Herstellung von Verbindungen gemäß Anspruch 4 durch Umsetzung von Verbindungen der Formel 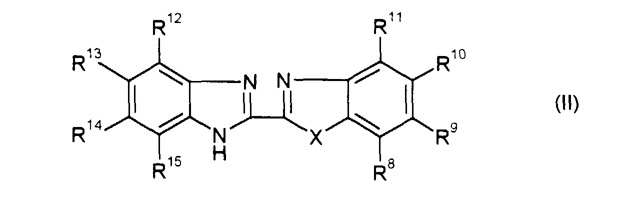 mit R¹-Halogeniden, wobei X und die Substituenten R⁸ bis R¹⁵ die in Anspruch 1 angegebenen Bedeutungen und der Substituent R¹ die in Anspruch 4 angegebene Bedeutung besitzen.

6. Verfahren zur Herstellung von Verbindungen gemäß Anspruch 4 durch Umsetzung von Verbindungen der Formel 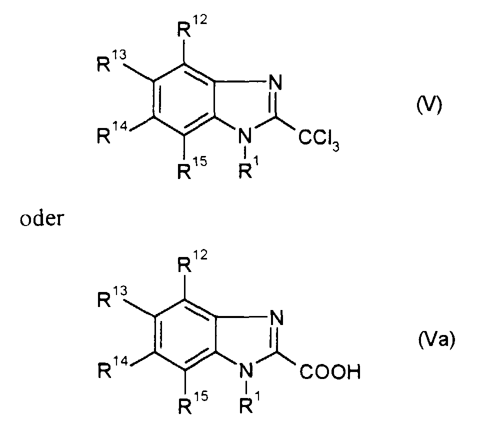 oder Carbonsäurehalogenide, -ester, -amide oder -nitrile der Säure Va
mit Verbindungen der Formel 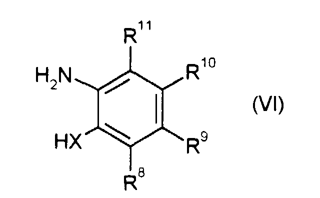 worin X und die Substituenten R⁸ bis R¹⁵ die in Anspruch 1 angegebenen Bedeutungen und der Substituent R¹ die in Anspruch 4 angegebene Bedeutung besitzen.

7. Verfahren zur Herstellung von Verbindungen gemäß Anspruch 4 durch Umsetzung von Verbindungen der Formel 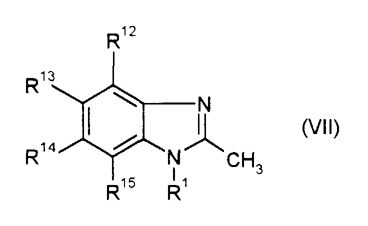 mit Verbindungen der Formel 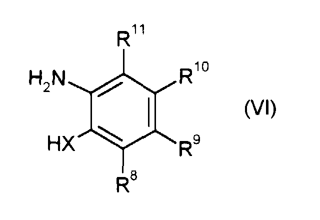 in Gegenwart von Schwefel, wobei X und die Substituenten R⁸ bis R¹⁵ die in Anspruch 1 angegebenen Bedeutungen und der Substituent R¹ die in Anspruch 4 angegebene Bedeutung besitzen.

8. Verfahren zur Herstellung von Verbindungen gemäß Anspruch 4, wobei X für Schwefel steht, durch Umsetzungen von Verbindungen der Formel 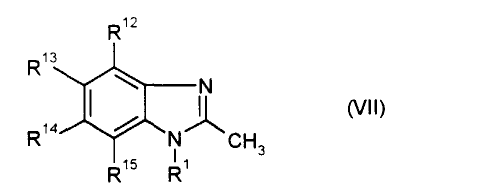 mit Verbindungen der Formel 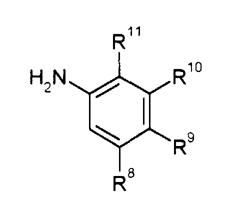 in Gegenwart von Schwefel, wobei die Substituenten R⁸ bis R¹⁵ die in Anspruch 1 angegebenen Bedeutungen und der Substituent R¹ die in Anspruch 4 angegebene Bedeutung besitzen.
